# EUROPEAN PATENT APPLICATION

(11) **EP 4 814 107 A1**
(43) Date of publication of application: **30.09.2026**
(21) Application number: 24894035.5
(22) Date of filing: 11.11.2024
(51) Int. Cl.: C12N 1/21, C12N 15/60, C12P 7/22

(54) **PHENOL-GENERATING HYDROGENOPHILUS BACTERIUM TRANSFORMANT**

(30) Priority: 21.11.2023 JP 2023197759
(71) Applicant: Utilization of Carbon Dioxide Institute Co.,Ltd., Tokyo 135-0091 (JP)
(72) Inventor: YUKAWA Hideaki, Tokyo 135-0091 (JP)
(74) Representative: Müller-Boré & Partner Patentanwälte PartG mbB
(86) International application number: PCT/JP2024/040016
(87) International publication number: WO 2025/110043

(57) **Abstract**

A transformant obtained by introducing a tyrosine phenol-lyase gene described in (a), (b), (c), (d), or (e) below into a *Hydrogenophilus* bacterium is capable of efficiently producing phenol utilizing substantially only carbon dioxide as a carbon source.
(a) A DNA comprising the nucleotide sequence of SEQ ID NO: 2 or 4
(b) A DNA comprising a nucleotide sequence that has 90% or more identity to SEQ ID NO: 2 or 4 and encoding a polypeptide having tyrosine phenol-lyase activity
(c) A DNA encoding a polypeptide comprising the amino acid sequence of SEQ ID NO: 3 or 5
(d) A DNA encoding a polypeptide comprising an amino acid sequence that has 90% or more identity to SEQ ID NO: 3 or 5, the polypeptide having tyrosine phenol-lyase activity
(e) A DNA encoding a polypeptide comprising an amino acid sequence that has 1 to 40 amino acids deleted, substituted, inserted, or added in the amino acid sequence of SEQ ID NO: 3 or 5, the polypeptide having tyrosine phenol-lyase activity

## Description

### Technical Field

The present invention relates to a *Hydrogenophilus* bacterium transformant having ability to produce phenol, and to a method for producing phenol using the same.

### Background Art

The Paris Agreement, which was adopted in 2015, provides that global emissions of greenhouse gases should be promptly reduced. The Sixth Assessment Report published in 2023 by the Intergovernmental Panel on Climate Change (IPCC) states that if greenhouse gas emissions continue at high levels, there is a very high likelihood that the global average temperature will rise by more than 1.5°C compared to the pre-industrial era during the period from 2021 to 2040, and even if the emissions are kept low, the possibility of exceeding 1.5°C remains. The report also indicates that there would be a clear difference in climate change arisen across regions of the world between in the case where the temperature increase is limited to 1.5°C and in the case where the temperature reaches 2°C. Based on this, the report asserts that carbon neutrality must be achieved, that is, the global net anthropogenic CO₂ emissions must be reduced by approximately 45% by 2030 compared to 2010 levels and must reach net zero around 2050. In accordance with this report, the significant reduction of greenhouse gas emissions, including carbon dioxide and methane, has become a shared global challenge, thereby increasing the necessity for the development of relevant technologies.

The materials currently supplied by the chemical industry are indispensable for maintaining the present standards of science, culture, and daily life around the world, and a continuous and stable supply is required. However, the majority of chemical production globally still relies on petroleum-based raw materials, which contributes to the increase in greenhouse gas emissions. Accordingly, chemical manufacturing methods are expected to shift from conventional processes that heavily consume petroleum resources to carbon recycling-based methods that make effective use of carbon resources. To this end, research and development of biorefineries, which produce green chemicals from biomass using microbial fermentation, is being actively pursued in various countries. However, the saccharification of biomass for use as raw materials of microbial fermentation necessitates complex processes, and therefore, be costly. Using biomass that could serve as food or feed for chemical production disturbs the stable supply of food and feed. Furthermore, the large-scale consumption of biomass for chemical manufacturing raises concerns that it actually leads to environmental destruction.

In research on carbon recycling aimed at reducing fossil fuel consumption and greenhouse gas emissions, gases such as carbon dioxide, methane, and carbon monoxide are attracting attention as carbon sources having a higher level of sustainability. If these gases present in the atmosphere can be fixed by microorganisms and utilized for industrial purposes, it would significantly contribute to carbon recycling and support the achievement of the targeted carbon neutrality. Thus, techniques for producing valuable chemicals and biofuels using microorganisms that grow by utilizing these gases are the subject of interest. In particular, carbon fixation and efficient utilization of carbon dioxide, a significant contributor to global warming, is highly anticipated.

Phenol is widely used as a raw material for producing resins such as phenolic resin and epoxy resin, dyes, synthetic fragrances, agrochemicals, surfactants, picric acid, salicylic acid, phenacetin, bisphenol A, aniline, p-phenolsulfonic acid, 2-phenoxyethanol, and other compounds. Phenol itself is also used as a sterilizing disinfectant, a dental anesthetic, a solvent, an analytical reagent, and the like.

In particular, phenolic resin is an important compound in high demand. Phenolic resin is the oldest synthetic resin and exhibits excellent properties such as heat resistance and durability. Phenolic resin is used as a raw material for automotive parts, electronic products such as semiconductor components and electronic circuit components, and other various products, and is also used across a wide range of fields, including healthcare, agriculture, and construction. Furthermore, phenolic resin is combined with other materials such as glass fibers and organic or inorganic fillers to create new functional materials, and the demand for phenolic resin continues to grow.

Currently, phenol is industrially produced using cumene method, which uses petroleum (coal tar)-derived benzene and propylene as raw materials. This method includes complex steps and is a high-temperature, high-pressure chemical industrial process, which consumes large amounts of energy. Therefore, there is a need to shift away from chemical synthesis processes that depend on petroleum resources.

Recently, microbial fermentation-based phenol production using renewable, non-edible biomass resources has attracted increasing attention as an approach to shift from a petroleum-dependent society, which faces the risk of future depletion, to a sustainable society. However, as mentioned above, the use of biomass poses various problems, such as complex processing requirements, disruption of the stable supply of food and feed, and a high environmental burden.

Therefore, there is a need for a practical method for producing phenol using microorganisms in a simpler process without disrupting the stable supply of food and feed or imposing an environmental burden. In particular, there is a need for a practical method that can produce phenol through carbon dioxide fixation.

Many microorganisms possess a metabolic pathway that produces phenol from the aromatic amino acid tyrosine via the catalytic action of tyrosine phenol-lyase and a metabolic pathway that produces phenol from 4-hydroxybenzoic acid or salicylic acid on the shikimate pathway via the catalytic action of decarboxylase. However, no microorganisms have been reported to actually produce phenol.

Techniques for producing phenol by fermentation with genetically modified microorganisms have been reported. Patent Literature 1 discloses the expression of an active polypeptide from a tyrosine phenol-lyase gene from *Pantoea agglomerans*, *Citrobacter braakii*, *Desulfitobacterium hafniense*, *Chloroflexus aurantiacus*, *Nostoc punctiforme*, or *Treponema denticola* introduced into *Corynebacterium glutamicum*, and in particular, a method for producing phenol from glucose as a carbon source using a transformant obtained by introduction of the tyrosine phenol-lyase gene from *Pantoea agglomerans*.

Non Patent Literature 1 to 4 teach methods for producing phenol from glucose using a transformant obtained by introducing a tyrosine phenol-lyase gene from *Pasteurella multocida* into *Escherichia coli* and methods for producing phenol from glucose using a transformant obtained by introducing a tyrosine phenol-lyase gene from *Pantoea agglomerans* into *Pseudomonas putida* or *Pseudomonas taiwanensis*.

Non Patent Literature 5 teaches a method for producing phenol from glucose by microbial co-culture of Escherichia coli transformed with a 4-hydroxybenzoate decarboxylase gene from *Escherichia coli* and *Escherichia coli* transformed with a tyrosine phenol-lyase gene from *Pasteurella multocida*.

Non Patent Literature 6 teaches a method for producing phenol from glucose using a transformant obtained by introducing a tyrosine phenol-lyase gene from *Citrobacter braakii*, a 4-hydroxybenzoate decarboxylase gene from *Klebsiella pneumoniae*, and a salicylate decarboxylase gene from *Trichosporon moniliiforme* into *Escherichia coli*.

However, these methods are those producing phenol by cultivating microorganisms using sugars, which are costly to produce, and none of the methods are those producing phenol using carbon dioxide as a carbon source.

### Citation List

### Patent Literature

[Patent Literature 1] WO2012/033112 A

### Non-Patent Literature

[Non Patent Literature 1] J Bacteriol., 190 (8): 2822-2830 (2008)
[Non Patent Literature 2] Appl Environ Microbiol., 71(12):8221-8227 (2005)
[Non Patent Literature 3] Metab Eng., 47 : 121- 133 (2018)
[Non Patent Literature 4] Biotechnol J., 9(5):621-629(2014)
[Non Patent Literature 5] Biotechnol Bioeng., 116(12):3349-3359(2019)
[Non Patent Literature 6] Fermentation., 7(4):216(2021)

### Summary of Invention

### Technical Problem

The objective of the present invention is to provide a transformant of a *Hydrogenophilus* bacterium that is capable of efficiently producing phenol utilizing carbon dioxide as a sole carbon source, and a method for efficiently producing phenol using this transformant.

### Solution to Problem

The inventor of the present invention has focused on *Hydrogenophilus* bacteria as bacteria capable of fixing carbon dioxide on an industrial scale for the purpose of avoiding the use of high cost raw materials such as sugars and contributing to global warming countermeasures. H*ydrogenophilus* bacteria are bacteria which grow by producing organic substances from carbon dioxide by utilizing hydrogen energy. The growth rate of such bacteria is generally extremely slow, however, the growth rate of *Hydrogenophilus* bacteria is fast, and their ability of fixing carbon dioxide is remarkably higher than that of plants and photosynthetic bacteria.

*Hydrogenophilus* bacteria do not have enzymes for producing phenol. Therefore, it is necessary to introduce the gene of an enzyme which catalyzes a reaction producing phenol to *Hydrogenophilus* bacteria in order to provide capability of producing phenol on an industrial scale to *Hydrogenophilu*s bacteria.

The inventor of the present invention has found that when a heterologous gene which is expressed within bacteria other than *Hydrogenophilus* bacteria is introduced into *Hydrogenophilus* bacteria, a functioning protein often is not produced or is insufficiently produced. It is generally not practical to divert a gene which can produce a substance within bacteria other than *Hydrogenophilus* bacteria to *Hydrogenophilus* bacteria in order to produce the substance.

Under such circumstances, the present inventor sought to obtain tyrosine phenol-lyase genes expressible in *Hydrogenophilus* bacteria and examined genes predicted to encode tyrosine phenol-lyase in the genomes of other microorganisms. The present inventor has finally found that the tyrosine phenol-lyase genes of *Chloroflexus aggregans* and *Chloroflexus aurantiacus* enable the expression of functional tyrosine phenol-lyase in *Hydrogenophilus* bacteria. The present inventor has also found that the transformants obtained by introducing these genes into *Hydrogenophilus* bacteria can efficiently produce phenol using carbon dioxide as a sole carbon source.

The present invention has been completed on the basis of the above findings and provides the following transformant and method for producing phenol.
[1] A transformant obtained by introducing a tyrosine phenol-lyase gene described in (a), (b), (c), (d), or (e) below into a *Hydrogenophilus* bacterium.
   (a) A DNA comprising the nucleotide sequence of SEQ ID NO: 2 or 4
   (b) A DNA comprising a nucleotide sequence that has 90% or more identity to SEQ ID NO: 2 or 4 and encoding a polypeptide having tyrosine phenol-lyase activity
   (c) A DNA encoding a polypeptide comprising the amino acid sequence of SEQ ID NO: 3 or 5
   (d) A DNA encoding a polypeptide comprising an amino acid sequence that has 90% or more identity to SEQ ID NO: 3 or 5, the polypeptide having tyrosine phenol-lyase activity
   (e) A DNA encoding a polypeptide comprising an amino acid sequence that has 1 to 40 amino acids deleted, substituted, inserted, or added in the amino acid sequence of SEQ ID NO: 3 or 5, the polypeptide having tyrosine phenol-lyase activity
[2] The transformant according to [1], wherein the *Hydrogenophilus* bacterium is *Hydrogenophilus thermoluteolus*.
[3] A method for producing phenol, the method comprising a step of culturing the transformant according to [1] or [2].

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical products can be produced while suppressing global warming attributed to increased carbon dioxide emissions.

Phenol is an industrially important aromatic compound. However, the process of chemical synthesis from petroleum feedstock is undesirable from the viewpoint of environmental protection. In contrast, the production of phenol using carbon dioxide-fixing microorganisms can provide a solution to global warming caused by increased carbon dioxide emissions as well as to securing the supply of industrially necessary phenol.

Bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, such bacteria grow slowly, but the growth rate of hydrogen-oxidizing bacteria, *Hydrogenophilus* bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

According to the present invention, introducing a specific tyrosine phenol-lyase gene into *Hydrogenophilus* bacteria enables the expression of functional tyrosine phenol-lyase within these bacteria to produce phenol on an industrial scale.

As described above, *Hydrogenophilus* bacteria have an atypically remarkable carbon dioxide fixation ability among organisms having carbon dioxide fixation ability. Therefore, the present invention has established a pathway for the industrial-scale production of phenol.

### Mode for Carrying Out the Invention

Hereinafter, the present invention will be described in detail.

### (1) Transformant capable of producing phenol

The transformant (transformed bacterium) of the present invention is a transformant obtained by introducing the tyrosine phenol-lyase gene (hereinafter sometimes abbreviated as "*tpl* gene") of *Chloroflexus aggregans* or *Chloroflexus aurantiacu*s or a homologue thereof into a host *Hydrogenophilus* bacterium. That is, the transformant of the present invention is a *Hydrogenophilus* bacterium transformant having an exogenous *tpl* gene, such as the *tpl* gene of *Chloroflexus aggregans* or *Chloroflexus aurantiacus*, or a homologue thereof.

The *tpl* gene used in the present invention does not need to have been previously identified as *tpl* gene and may be any DNA encoding a polypeptide having tyrosine phenol-lyase activity. Tyrosine phenol-lyase activity refers to an enzymatic activity that converts tyrosine to phenol.

In the present invention, the *tpl* gene may be DNA isolated from naturally occurring bacteria and encoding tyrosine phenol-lyase, or may be DNA artificially synthesized using methods known to those skilled in the art.

The transformant (transformed bacterium) of the present invention needs only to be a *Hydrogenophilus* bacterium into which the *tpl* gene of *Chloroflexus aggregans* or *Chloroflexus aurantiacus* or a homologue thereof has been introduced, and may encompass a transformant obtained by introducing two or more of these genes into a Hydrogenophilus bacterium and a transformant obtained by introducing not only these genes but also another gene or other genes into a Hydrogenophilus bacterium.

### Tyrosine phenol-lyase gene (tpl gene)

In the present invention, a DNA comprising the nucleotide sequence of SEQ ID NO: 2 or 4 (in particular, a DNA consisting of the nucleotide sequence of SEQ ID NO: 2 or 4) can be used as *tpl* gene. SEQ ID NO: 2 represents the nucleotide sequence of the *tpl* gene of *Chloroflexus aggregans*, and SEQ ID NO: 4 represents the nucleotide sequence of the *tpl* gene of *Chloroflexus aurantiacus*.

In the present invention, a DNA comprising a nucleotide sequence that has 90% or more, even 95% or more, even 98% or more, even 99% or more identity to SEQ ID NO: 2 or 4 (in particular, a DNA consisting of a nucleotide sequence that has 90% or more, even 95% or more, even 98% or more, even 99% or more identity to SEQ ID NO: 2 or 4) and encoding a polypeptide having tyrosine phenol-lyase activity can also be used.

In the present invention, a DNA encoding a polypeptide comprising the amino acid sequence of SEQ ID NO: 3 or 5 (in particular, a polypeptide consisting of the amino acid sequence of SEQ ID NO: 3 or 5) can also be used as *tpl* gene. SEQ ID NO: 3 represents the amino acid sequence of the tyrosine phenol-lyase of *Chloroflexus aggregans*, and SEQ ID NO: 5 represents the amino acid sequence of the tyrosine phenol-lyase of *Chloroflexus aurantiacus*.

In addition, a DNA encoding a polypeptide comprising an amino acid sequence that has 90% or more, even 95% or more, even 98% or more, even 99% or more identity to SEQ ID NO: 3 or 5 (in particular, a polypeptide consisting of an amino acid sequence that has 90% or more, even 95% or more, even 98% or more, even 99% or more identity to SEQ ID NO: 3 or 5) and having tyrosine phenol-lyase activity can be used.

Furthermore, a DNA encoding a polypeptide comprising an amino acid sequence that has 1 to 40, 1 to 5, 1 to 4, 1 to 3, or 1 to 2 amino acids, or 1 amino acid deleted, substituted, inserted, or added in the amino acid sequence of SEQ ID NO: 3 or 5 (in particular, a polypeptide consisting of an amino acid sequence that has 1 to 40, 1 to 5, 1 to 4, 1 to 3, or 1 to 2 amino acids, or 1 amino acid deleted, substituted, inserted, or added in the amino acid sequence of SEQ ID NO: 3 or 5) and having tyrosine phenol-lyase activity can be used.

In view of codon degeneracy or codon preference in *Hydrogenophilus* bacteria, the DNA sequence encoding a protein comprising the amino acid sequence of SEQ ID NO: 3 or 5 may be subjected to a variety of nucleotide substitutions in the coding region, as long as such substitutions do not alter the amino acid sequence of the protein expressed from the coding region.

In the present invention, the identities of nucleotide sequences and amino acid sequences were calculated using GENETYX ver.17 (made by GENETYX Corporation).

In the present invention, the presence of tyrosine phenol-lyase activity in a polypeptide of interest is determined by reacting the polypeptide with tyrosine and detecting the resulting phenol by high-performance liquid chromatography (HPLC).

In the present invention, a DNA comprising a nucleotide sequence that has 90% or more but less than 100% identity to the nucleotide sequence of a given DNA and encoding a polypeptide having the same type of activity as the polypeptide encoded by the given DNA is called a "homolog" of the given DNA. A DNA encoding a polypeptide comprising an amino acid sequence that has 90% or more but less than 100% identity to the amino acid sequence of a given polypeptide and having the same type of activity as the given polypeptide is called a homolog of the DNA encoding the given polypeptide. A DNA encoding a polypeptide comprising an amino acid sequence that has 1 to 40 amino acids deleted, substituted, inserted, or added in the amino acid sequence of a given polypeptide and having the same type of activity as the given polypeptide is also called a homolog of the DNA encoding the given polypeptide.

### Hydrogenophilus bacteria

Examples of *Hydrogenophilus* bacteria include *Hydrogenophilus thermoluteolus*, *Hydrogenophilus halorhabdus*, *Hydrogenophilus denitrificans*, *Hydrogenophilus hirschii*, *Hydrogenophilus islandicus*, *Hydrogenophilus thiooxidans, Hydrogenophilus* bacterium strain Mar3 (*Hydrogenophilus* sp. Mar3), and *Hydrogenophilus* bacterium strain Z1038 (*Hydrogenophilus* sp. Z1038). In particular, *Hydrogenophilus thermoluteolus* is preferable because its superior growth rate enables top-level carbon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977))(It proliferates double per hour.). *Hydrogenophilus thermoluteolus* strain NBRC 14978 is internationally deposited under the Budapest Treaty, and is thus publicly available.

The hosts *Hydrogenophilus* bacteria may be bacteria isolated from nature or genetically modified bacteria derived from bacteria isolated from nature. Genetic modification may be intended, for example, to allow high expression of an introduced *tpl* gene.

Such a genetic modification can be made, for example, by removing (curing) endogenous plasmids in *Hydrogenophilus* bacteria, disruption of genes on the genome of *Hydrogenophilus* bacteria, or other techniques. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Method for producing transformant

Next, methods for obtaining transformants by introducing *tpl* gene into *Hydrogenophilus* bacteria are described.

The introduction of *tpl* gene into *Hydrogenophilus* bacteria can be carried out using common methods for introducing exogenous genes into bacteria. The *tpl* gene may be directly introduced into *Hydrogenophilus* bacteria. Alternatively, it may be incorporated into a vector for transformation such as a plasmid vector, viral vector, cosmid, fosmid, or BAC, and then, an obtained vector may be introduced into *Hydrogenophilus* bacteria.

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like based on its DNA sequence.

Among them, pCAMO-6 (SEQ ID NO: 1) is preferable. Those skilled in the art can prepare pCAMO-6 by utilizing a gene synthesis service or the like.

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of *tpl* gene into a *Hydrogenophilus* bacterium (transformation) can be carried out by publicly known methods such as calcium chloride method, calcium phosphate method, rubidium chloride method, and electric pulse method (electroporation method).

### (2) Method for producing phenol

The present invention provides a method for producing phenol with use of the transformant of the present invention described above. The method includes a step of culturing the transformant of the present invention, especially, a step of culturing the transformant of the present invention using an inorganic or organic culture medium while supplying a gas containing carbon dioxide, preferably a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that phenol can be produced efficiently.

*Hydrogenophilus* bacteria can grow using hydrogen as a source of energy and using carbon dioxide as a sole carbon source, and thus, *Hydrogenophilus* bacteria can fix carbon dioxide efficiently when phenol is produced using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using only carbon dioxide as a carbon source" encompasses cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and phenol can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, mixed gases can be continuously supplied into an airtight culture container and shaking culture can be carried out, or the transformant can be cultured using an airtight culture container while introducing mixed gases into the culture medium by bubbling.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and phenol can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and phenol can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and phenol can be produced efficiently.

Phenol is produced in the medium solution by culturing the transformant in the above-described manner. Phenol can be recovered by collecting obtained medium solution. Further, phenol can be separated from the medium solution by known methods such as distillation, membrane permeation, and organic solvent extraction.

### Examples

The present invention will now be described with reference to examples. The technical scope of the present invention is not limited by the following examples.

### (1) Methods for PCR, electrophoresis, DNA recovery, and seamless cloning in the construction of expression plasmids

PCR, electrophoresis, DNA recovery, and the preparation of plasmid vector pCAMO-6 for seamless cloning in the construction of marker cassettes and expression plasmids were performed using the methods described below.

### (1-1) PCR, electrophoresis, and DNA recovery

PCR was performed in the usual manner using DNA Thermal Cycler manufactured by Life Technologies Inc. and using KOD One PCR Master Mix (manufactured by Toyobo Co., Ltd.) as a reaction reagent. After that, the resulting reaction mixture was subjected to electrophoresis on a 1% agarose gel. Appropriate DNA fragments were recovered from the gel using GEL/PCR Purification Mini Kit (FAVORGEN) as needed.

### (1-2) Preparation of plasmid vector pCAMO-6 for seamless cloning

For seamless cloning, plasmid vector pCAMO-6 was amplified by PCR using the DNA of SEQ ID NO: 1 as a template. The primers shown below were used for PCR.

### Primers for amplification of plasmid vector pCAMO-6

(a-1) 5'-GAATTCGAGCTCCGTCGACA-3' (SEQ ID NO: 6)
(b-1) 5'-ATGCGTTTCTCCTCCAGATC-3' (SEQ ID NO: 7)

The electrophoresis results showed that an approximately 5.2-kbp DNA fragment corresponding to the vector gene was detected. The DNA fragment was recovered from the gel.

### (1-3) Joining of vector pCAMO-6 and insert DNA fragments

The DNA fragment containing vector pCAMO-6 synthesized above was joined to each insert DNA fragment in a one-to-one manner by the recombinase extracted from Escherichia coli strain JM109.

Escherichia coli strain JM109 was transformed separately with each resulting reaction mixture by heat shock method, applied to LB medium containing 50 µg/mL kanamycin, and cultured at 37°C for 24 hours.

### (1-4) Plasmid extraction and gene sequence confirmation

Strains grown on the LB medium were individually inoculated into 5 mL of liquid LB medium containing 50 µg/mL kanamycin per test tube using a platinum loop, and cultured with shaking at 37°C. Plasmid DNA was extracted from each culture liquid. The analysis of the sequences of the genes inserted in the plasmids was outsourced to Eurofins Genomics K.K. and performed by Sanger method. The results confirmed that the gene sequences were identical to the corresponding sequences in a database.

### (2) Construction of tpl gene expression plasmids

DNA fragments consisting of the *tpl* genes from different bacteria shown below were amplified by PCR.
*Chloroflexus aggregans* (SEQ ID NO: 2)
*Chloroflexus aurantiacus* (SEQ ID NO: 4)
*Chryseobacterium gleum* (SEQ ID NO: 8)
*Serratia plymuthica* (SEQ ID NO: 9)

The primers shown below were used for PCR.

### Primers for amplification of Chloroflexus aggregans tpl gene

(a-2) 5'-CTGGAGGAGAAACGCATATGGAGATGGAACCAGACTTCCCAC-3' (SEQ ID NO: 10)
(b-2) 5'-CGACGGAGCTCGAATTCTCACTCCGTAACCGGTTCAAAGCGGGC-3' (SEQ ID NO: 11)

The primers (a-2) and (b-2) contain sequences homologous to regions of vector pCAMO-6.

### Primers for amplification of Chloroflexus aurantiacus tpl gene

(a-3) 5'-CTGGAGGAGAAACGCATATGCAGGAACAAGACTACCCCCGTAC-3' (SEQ ID NO: 12)
(b-3) 5'-CGACGGAGCTCGAATTCTCATTCCACCGGTTCAAACCGGGCCTG-3' (SEQ ID NO: 13)

The primers (a-3) and (b-3) contain sequences homologous to regions of vector pCAMO-6.

### Primers for amplification of Chryseobacterium gleum tpl gene

(a-4) 5'-CTGGAGGAGAAACGCATATGAATTTACCGTACGCGGAAC-3' (SEQ ID NO: 14)
(b-4) 5'-CGACGGAGCTCGAATTCTTAGGCTTTTTCAAGCTGAACAG-3' (SEQ ID NO: 15)

The primers (a-4) and (b-4) contain sequences homologous to regions of vector pCAMO-6.

### Primers for amplification of Serratia plymuthica tpl gene

(a-5) 5'-CTGGAGGAGAAACGCATATGTCCTACCCAGCTGAACC-3' (SEQ ID NO: 16)
(b-5) 5'-CGACGGAGCTCGAATTCTCACAAATATTCAAAGCGCGCG-3' (SEQ ID NO: 17)

The primers (a-5) and (b-5) contain sequences homologous to regions of vector pCAMO-6.

The electrophoresis results showed that approximately 1.4-kbp DNA fragments corresponding to the *tpl* genes from the respective bacterial strains were detected. The DNA fragments were recovered from the agarose gel.

The DNA fragments containing the *tpl* genes were individually joined to the DNA fragment containing vector pCAMO-6, and Escherichia coli strain JM109 was transformed separately with each resulting plasmid. After that, the plasmids were extracted, and the sequences of the *tpl* genes were confirmed.

### (3) Transformants of Hydrogenophilus thermoluteolus

### (3-1) Gene transfer

*Hydrogenophilus thermoluteolus* strain TH-1 was transformed separately with each plasmid obtained in the section "(2) Construction of *tpl* gene expression plasmids" by electric pulse method (electroporation), applied to solid LB medium containing 50 µg/mL kanamycin, and cultured at 52°C for 24 hours. Strains grown on the solid LB medium were individually inoculated onto solid LB medium containing 50 µg/mL kanamycin using a platinum loop, and cultured at 52°C for 24 hours. PCR was performed on the grown strains on the solid LB medium to confirm amplification of the fragments inserted in the plasmids. PCR was performed in the usual manner using DNA Thermal Cycler manufactured by Life Technologies Inc. and using KOD One PCR Master Mix (manufactured by Toyobo Co., Ltd.) as a reaction reagent.

The results showed that DNA fragments of approximately 1.4 kbp in length corresponding to the respective *tpl* genes were amplified. The plasmids containing the *tpl* genes from the respective bacteria, as well as the transformants of *Hydrogenophilus thermoluteolus* strain TH-1 with these plasmids, were named as shown in Table 1.

### (3-2) Phenol production

The transformed strains with the *tpl* genes prepared as described above were individually inoculated into liquid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, and 4.0 mg of CoCl₂·6H₂O were dissolved in 1 L of distilled water (pH 7.0)] containing 50 µg/mL kanamycin using a platinum loop, and cultured with shaking at 52°C for 72 hours while a gas mixture of H₂:O₂:CO₂ = 7.5:1:1.5 was supplied. Similarly, *Hydrogenophilus thermoluteolus* strain TH-1 transformed with empty vector pCAMO-6 without any *tpl* gene was cultured. After that, culture supernatants were collected by centrifugation (4°C, 5,000 g, 10 minutes). The concentration of phenol in each culture supernatant was determined by high-performance liquid chromatography (Shimadzu Corporation) under the following conditions.
Mobile phase A: 0.1% aqueous phosphate solution
Mobile phase B: 50% acetonitrile
Flow rate: 1 mL/min
Column: CAPCELL PAK MG II, 5.0 µm, 4.6 mm I.D. × 150 mm
Column temperature: 40°C
PDA temperature: 40°C
PDA: 200 to 300 nm
Reference wavelength: 210 nm

As shown in Table 1, 0.3 mM phenol was detected in the culture supernatant of transformed strain Tpl01 carrying the *tpl* gene of *Chloroflexus aggregans*. In addition, 0.15 mM phenol was detected in the culture supernatant of transformed strain Tpl02 carrying the *tpl* gene of *Chloroflexus aurantiacus*. In contrast, no phenol was detected in the transformed strain carrying the *tpl* gene of *Chryseobacterium gleum* or *Serratia plymuthica*, or the strain carrying no *tpl* gene.

**[Table 1]**

| Strain | Origin of tyrosine phenol-lyase gene | Concentration of phenol in medium supernatant(mM) |
|---|---|---|
| Tpl01 | *Chloroflexus aggregans* | 0.3 |
| Tpl02 | *Chloroflexus aurantiacus* | 0.15 |
| Tpl03 | *Chryseobacterium gleum* | ND |
| Tpl04 | *Serratia plymuthica* | ND |
| p-CAMO-6/TH-1 | None | ND |

### ∺ND: Not Detected

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

SEQ ID Nos: 1, 2, 3, 4, 5, 8, and 9 are shown below.
[Chem. 1]
   The first half of SEQ ID NO: 1 (pCAMO-6) 
cccatcgactgcacggtgcaccaatgcttctggcgtcaggcagccatcggaagctgtggtatggctgtgcaggt
cgtaaatcactgcataattcgtgtcgctcaaggcgcactcccgttctggataatgttttttgcgccgacatcat
aacggttctggcaaatattctgaaatgagctgttgacaattaatcatcggctcgtataatgtgtggaattgtga
gcggataacaatttcacacaggaaacaggccagttaggcctgctagcagatctggaggagaaacgcatatgctg
atatcggatccgaattcgagctccgtcgacaagcttgcggccgcactgcagcaccatcaccaccatcattgata
agatccggctgctaacaaagcccgaaaggaagctgagttggctgctgccaccgctgagcaataactcgaggcca
atgaggccttcaaataaaacgaaaggctcagtcgaaagactgggcctttcgttttatctgttgtttgtcggtga
acgctctcctgagtaggacaaatccgccgggagcggatttgaacgttgcgaagcaacggcccggagggtggcgg
gcaggacgcccgccataaactgccaggcatcaaattaagcagaaggccatcctgacggatggcctttttgcgtt
tctacaaactccgcggatctaggtgaagatcctttttgataatctcatgaccaaaatcccttaacgtgagtttt
cgttccactgagcgtcagaccccgtagaaaagatcaaaggatcttcttgagatcctttttttctgcgcgtaatc
tgctgcttgcaaacaaaaaaaccaccgctaccagcggtggtttgtttgccggatcaagagctaccaactctttt
tccgaaggtaactggcttcagcagagcgcagataccaaatactgtccttctagtgtagccgtagttaggccacc
acttcaagaactctgtagcaccgcctacatacctcgctctgctaatcctgttaccagtggctgctgccagtggc
gataagtcgtgtcttaccgggttggactcaagacgatagttaccggataaggcgcagcggtcgggctgaacggg
gggttcgtgcacacagcccagcttggagcgaacgacctacaccgaactgagatacctacagcgtgagctatgag
aaagcgccacgcttcccgaagggagaaaggcggacaggtatccggtaagcggcagggtcggaacaggagagcgc
acgagggagcttccagggggaaacgcctggtatctttatagtcctgtcgggtttcgccacctctgacttgagcg
tcgatttttgtgatgctcgtcaggggggcggagcctatggaaaaacgccagcaacgcggcctttttacggttcc
tggccttttgctggccgcggacgtagaaagccagtccgcagaaacggtgctgaccccggatgaatgtcagctac
tgggctatctggacaagggaaaacgcaagcgcaaagagaaagcaggtagcttgcagtgggcttacatggcgata
gctagactgggcggttttatggacagcaagcgaaccggaattgccagctggggcgccctctggtaaggttggga
agccctgcaaagtaaactggatggctttcttgccgccaaggatctgatggcgcaggggatcaagatccgatcaa
gagaggccactaaggcccaggatgaggatcgtttcgcatgattgaacaagatggattgcacgcaggttctccgg
ccgcttgggtggagaggctattcggctatgactgggcacaacagacaatcggctgctctgatgccgccgtgttc
cggctgtcagcgcaggggcgcccggttctttttgtcaagaccgacctgtccggtgccctgaatgaactacagga
cgaggcagcgcggctatcgtggctggccacgacgggcgttccttgcgcagctgtgctcgacgttgtcactgaag
cgggaagggactggctgctattgggcgaagtgccggggcaggatctcctgtcatctcaccttgctcctgccgag
aaagtatccatcatggctgatgcaatgcggcggctgcatacgcttgatccggctacctgcccattcgaccacca
agcgaaacatcgcatcgagcgagcacgtactcggatggaagccggtcttgtcgatcaggatgatctggacgaag
agcatcaggggctcgcgccagccgaactgttcgccaggctcaaggcgcgtatgcccgacggcgaggatctcgtc
gtgactcatggcgatgcctgcttgccgaatatcatggtggaaaatggccgcttttctggattcatcgactgtgg
ccggctgggtgtggcggaccgctatcaggacatagcgttggctacccgtgatattgctgaagagcttggcggcg
aatgggctgaccgcttcctcgtgctttacggtatcgccgctcccgattcgcagcgcatcgccttctatcgcctt
cttgacgagttcttctgaggccatctaggccatgagtaagtctgacggaacggaaagttgtcggttaccagcaa
cgtttcgccctgttggcctcaaaagttcgagtccgttattggtatttccataccaactcaaacgggatataatg
[Chem. 2]
   The latter half of SEQ ID NO: 1 (pCAMO-6) 
tgacatgtaacatttctcgattgaggaacgacaatgacagtaccggtcaaagcgcgtgttcaaaagtatcgcga
cgcgctgcgccgctcgggtttgcgtccggttcagatatgggtgccggatacccgccgcccgggctttgcacaag
agtgtcgccgccagtgcctgctcgtgacccaggcagacgccgctgatacgcaactccaacgactcatggatgaa
gccttggcagagattgatggctggcaggcatgatgcgcggcgatttggtaacgatcgttacccagggcgacttt
ggcaaaccaaggccagcgttggtgatccagtccgatctgttcaacgagctcagcagcataaccgtctttcctgt
aaccagtaccattgtggatgccccgttgttgcgcgtcacggttgagccaaccccggaaaacggcttgcacaagc
gatcacaggtgatggtggacaaaattatgaccgtgcggcgggacaaagtcggtcagatcattggacgtatcgat
acgactacgctgacaacgatcgagcgctgcttagctgtcttcttgggaatcgcgaaataaggccatgaaggcct
tacccatcaccgtgcatcacgccgaccgcttcgccactttgcgacaagaaagcaatctgaacgcgctggcgaaa
atggatcggcgctgcccggtctacctgtgaagttcgcgcaaacgtccatcgagacgtacattgccgttggcatc
gatctcgacggcgatttcctctggctaccgatcgcgcgcaccgcttacgcgccctcgacgtagtcgagaaaagc
gtgattgcgtatgttgcgccaactgagcccgtcggtgcgttgtgaccactcgttgtagaccacgcccaaaacca
tcaccccaaacggtgcggaaagtgctactctttcgtcttgccacaacaagaaggagggcgggtggtgatcgaaa
ccggtgctttgagagccaaagaagaccccattgcctgggtccaacagaaagcagccgaggcgttgcaacgccta
gatcaaaagatggcctctcagcccgtgcagatgtacctaccggggttcgatctaggggcctttcccaaccatgt
gaaccgctcctcgctctttgcgcctattgcgcgcggtcgccgcaaatttcatcgacaaacgaagatggtcagcc
gctcggatgctgtgatcgaatacactggagagcagctcgacgaagccgactgcgacctcatgatggcgctcatc
tggtttgcccagaaaaaaccgttaggggagtgggtgacattgaaccgagcgcagctgttgcggcggatcgggcg
cgcgacgggcaagcagaactacgagtggctccatcgcagaatgcgggcgatgaccgaagcgacactctacatcg
aagtgcgcaaatccgacggcagcacacgctacaagatcggctacgccgaggcgtttcacctcatcagcggcttt
gcgttcgatgagaaaagcgacgaatactgctaccaactcgatccccgctgggtacagctcttcagcaaccgcga
atacagcctgatcaacttcgacttgcgattgcagatcaaacggggtcaggacatggcgaaaaccctgcaacgcc
tcgttgctacttcttccgaccccatccagtcttacgaactcgaatggctcaagagcaaaatggaatacaccgga
agaatgcgcgatttcaaagagtcgctctccaaggcgtgcaatgagctgaaacggttgcacatcgtcagtgaata
caagatcactctggacaaaaagaagcaacgggagctgttgaagctctggattcccaaagcgatcacggcataaa
agcggtgcgacactgcacgatcagaaagtattttcagttactcaaacaccgttttttgttgaaaaacaacgaat
tgtcatcggtatagcgtcgcgcacccgagacgaccgacggtgtctggccatcggtatagcgtcgcgctttctcc
acctcttgaacagtcgttcggcatcggtatagcgtcgcgcctgcatcggtatagcgtcgcgctttttcggtata
gcgtcgcgctttttcggtatagcgtcgcgcggttgtcaaaaaatctattattattcaataagttacaagcggtt
tttgaccgtctactatatttctacttatttctactaggaagcgcctgtggataacttcgctcttttctacacag
cccctcagcggcgcaagcgcgccgctgggtagaaaattcctatcgcaacgaactgtcgttcctaaaaataggct
actcggtaaaacaaccgtataaatgatgccgcgtgctcggagtcaactccagaacggcatcggtatagtgtcgc
acttagcaatgtgcgaatatttcatcgttgagccgacttttggagattggtcatgggagatcctcgggttgtcg
aagccttggactcctgttgatagatccagtaatgacctcagaactccatctggatttgttcagaacgctcggtt
gccgccgggcgttttttattggtgagaatccagtctaga
[Chem. 3]
   SEQ ID NO: 2 (*tpl* gene of *Chloroflexus aggregans*) 
atggagatggaaccagacttcccacgcacgatgggtcagcagtttggccgccggtcgtgggccgagccgtggaa
gatcaagacggtcgagccgctgcggatcattagccgggccgagcgcgcggcagcgctgaaagccgccggctaca
acacctttttactccgttcggaagatgtttatatcgatctgctcaccgatagcggcaccaatgctatgagcgac
cggcagtgggcagcgttgatgatgggtgacgaggcgtatgccggtagccgcagtttctatcggttggaagcggc
agtgcagcaggcttacggctatcgtcatgtcattcccacccaccaaggccgtggcgccgagcatctgatcagcc
ggatcgctattcaacccggtcagtatgtgcccggtaatatgtatttcactactacccgtctccaccaagaactc
gccggtggcatatttgttgatgtgattatcgatgaagctcacgatccgcagagccaatatcccttcaaggggaa
tgtcgatctcgataagctgcaaacgctgatcaatcaagttggcgcaaaacagattgcctacgtcagcctcgccg
gtacggtcaatatggccggcggccagccggtcagtatggcgaacgtgcgcgctttgcgcgaattgtgcgaccgc
tacggcatccgcatttttctcgacgcgacccggttggttgagaatgccttcttcatcaaagagcgcgaacccgg
ctacgcgaatcatactatcgccgaaatcgtgcgcgagttttgtagctataccgacggtgcgtggatgagcgcca
aaaaggatagtctggtcaacatcgggggctggctggcgctgaacgacgatcagcttgccgatgaggcgcgcaat
ctggtggtggtgtacgaggggttgcatacctacggcgggatggccgggcgcgatatggaagcattggcagttgg
aattgaggaatcattacaagaggactacattcgggcgcgaatcggccaagtgcgctaccttggcgaactgctgc
tcgattggaatatcccgattgtcgtgccgatcggtgggcacgcgattttcctcgatgctcgccgtttctaccca
cacctcccccaagacctcttccctgcccaaactttagccgctgagctgtacctcgattcgggggtacgggcaat
ggaacgtggtatcgccagtgccggacgcgatcctaagaccgggcagcaccactatcccaaactcgaactgaccc
gcctcaccatcccacgccgagtctatacccaagcccacatggacgtagttgccgaatcggtgaagtcggtctat
gaccaacgtgaacgcgcccgtgggctgcgtatggtttatgagccgcgttacctgcgcttcttccaagcccgctt
tgaaccggttacggagtga
[Chem. 4]
   SEQ ID NO: 3 (tyrosine phenol-lyase of *Chloroflexus aggregans*) 
MEMEPDFPRTMGQQFGRRSWAEPWKIKTVEPLRIISRAERAAALKAAGYNTFLLRSEDVYIDLLTDSGTN
AMSDRQWAALMMGDEAYAGSRSFYRLEAAVQQAYGYRHVIPTHQGRGAEHLISRIAIQPGQYVPGNMYFT
TTRLHQELAGGIFVDVIIDEAHDPQSQYPFKGNVDLDKLQTLINQVGAKQIAYVSLAGTVNMAGGQPVSM
ANVRALRELCDRYGIRIFLDATRLVENAFFIKEREPGYANHTIAEIVREFCSYTDGAWMSAKKDSLVNIG
GWLALNDDQLADEARNLVVVYEGLHTYGGMAGRDMEALAVGIEESLQEDYIRARIGQVRYLGELLLDWNI
PIVVPIGGHAIFLDARRFYPHLPQDLFPAQTLAAELYLDSGVRAMERGIASAGRDPKTGQHHYPKLELTR
LTIPRRVYTQAHMDVVAESVKSVYDQRERARGLRMVYEPRYLRFFQARFEPVTE
[Chem. 5]
   SEQ ID NO: 4 (*tpl* gene of *Chloroflexus aurantiacus*) 
atgcaggaacaagactacccccgtacaatggggcaacaattcggtcggcggtcgtgggccgagccgtggaagat
caagatggttgagccgctgcgcgtgaccagccgggccgaacgcgaggcggcgctgaaggctgccggttacaaca
cgtttctgctgcgttctgaagatgtctatatcgatctgcttaccgatagtggtaccaatgccatgagcgaccgg
caatgggcagccctgatgatgggcgacgaggcatacgccgggagccgcagtttttatcgcctggaagcaactgt
ccaacaggtgtatggctaccgccacattattcccacccatcaggggcggggcgccgagcatctgatcagtcagg
tcgctatccgccgtgggcagtatgttcccggcaatatgtatttcacaaccacccgcctgcaccaggagctggcc
ggtggcatctttgttgatgtgattattgacgaagcgcacgatccccaaagccagtatccgtttaaaggcaacgt
cgatctcgacaaactacaggcgctgattgataaggttggcccgcaacagattgcctatatcagtctggccggta
ccgtcaacatggctggtgggcagccggtcagtatggctaacgtccgtgccttacgcgcattatgtgatcggtac
gggttgcgcatctttctcgattccacacgcttggttgagaatgcctttttcatcaaagaacgtgaacccggcta
tgccgaacaaagaatcgccgcgattgtccgcgagttttgcagttacaccgatggcgcatggatgagcgcaaaga
aggacgcgctggtgaacatcggtggctggttagcgctcaacgatgatcaactcgccgatgaagcccgcaatctg
gtggtggtgtacgaagggttgcacacctacggcggcatggccgggcgtgatatggaggcgctggcggtcgggat
tgaggagtcgctgcaagaggattacatccgtgcccgcatcggtcaggtgcgctacctcggcgaactgctcctcg
actgggacatccccatcgtagttccgattggcggtcacgcgatctttctggatgcacgccggttctatccgcac
ctgccgcaagacctcttccctgcccagaccctggccgccgagttgtacctcgattcaggggtgcgggctatgga
acgcggtattgccagcgccggacgcgatcccaagaccgggcagaactactatcccaaactggaattaacccggc
tgaccatcccgcgccgtgtttatactcaggcccacatggatgttgtggccgagtcggtgaaggcagtgtacgat
caacgtcatcaggcccgtggcctgcggatggtctacgaaccacggtacctccgcttcttccaggcccggtttga
accggtggaatga
[Chem. 6]
   SEQ ID NO: 5 (tyrosine phenol-lyase of *Chloroflexus aurantiacus*) 
MQEQDYPRTMGQQFGRRSWAEPWKIKMVEPLRVTSRAEREAALKAAGYNTFLLRSEDVYIDLLTDSGTNA
MSDRQWAALMMGDEAYAGSRSFYRLEATVQQVYGYRHIIPTHQGRGAEHLISQVAIRRGQYVPGNMYFTT
TRLHQELAGGIFVDVIIDEAHDPQSQYPFKGNVDLDKLQALIDKVGPQQIAYISLAGTVNMAGGQPVSMA
NVRALRALCDRYGLRIFLDSTRLVENAFFIKEREPGYAEQRIAAIVREFCSYTDGAWMSAKKDALVNIGG
WLALNDDQLADEARNLVVVYEGLHTYGGMAGRDMEALAVGIEESLQEDYIRARIGQVRYLGELLLDWDIP
IVVPIGGHAIFLDARRFYPHLPQDLFPAQTLAAELYLDSGVRAMERGIASAGRDPKTGQNYYPKLELTRL
TIPRRVYTQAHMDVVAESVKAVYDQRHQARGLRMVYEPRYLRFFQARFEPVE
[Chem. 7]
   SEQ ID NO: 8 (*tpl* gene of *Chryseobacterium gleum*) 
atgaatttaccgtacgcggaacctttccgcatcaaaatggtggaagaaatctaccagtctaccagagaagaaag
agagcagtggcttaaagaagctaattataaccttttcaatttaagatcttctcaggtttatattgacctgctta
ctgattccggaaccggagcaatgtccgataaacaatgggctgctttaatgacaggagatgaaagctacgcaggc
tcacgttcattcgaacaattacagaaaaccgttgaaagaattacaggattcaaatatttattaccaacccacca
gggaagagccgctgaaaacgtgcttttttccgtattggtaaaagaaggtgatgtagttccgggaaactcacact
ttgacactacaaaaggtcatatcgagttcagaaaggcacatgcaatagattgtacgattgatgaagcttttgac
atcaatgaccttcatcctttcaaagggaatatcaaccttgaaaaactggaagaagtttataaaagtcatcctaa
agaaagtattcctttctgtctgattaccattacctgtaactcatcaggaggacagcctgtttctctggaaaata
tgaaagctgtgaaagccctttctgatcaatatggaattcctgtattctttgattcagcgagatttgcagagaac
gcctatttcatcaaaaaaagagaggcaggacaggaaaacagaagcattaaagacatctgtaaggaaattttctc
ttacggagacggaatgacaatgagttccaaaaaagacgggctggtaaacattggtggattcatcgctttgaata
atgaagaagtgttcagaaaggcatccaactttaccattatctacgaaggctttattacttatggtggaatggcc
ggaagagatatggcagcactggctgtaggtctggacgaagcgactgagtttgcttatcttgaaagcagaatctc
ccaggttgaatatcttgggaataaactgatcgaatatggaattcctgttcagaaaccaatcgggggacacgccg
tatttattgattctttaaatttccttccgaatgtttcccgtgaagaatatccagcgcagacacttggtcttgaa
atttataaagaagcaggaatcagaactgtagaaatcgggactttattagcagacagagatcctgcaacaaggga
aaaccgttacccgaaattagaactggtacgtctcgcaattccaagaagaacttacaccaataatcatatggatt
atattgctgctgctataaagaacgtttatgaaagacgtgaagagattgcaaaagggtataagatcacctgggaa
cccgaaatcttaagacattttactgttcagcttgaaaaagcctaa
[Chem. 8]
   SEQ ID NO: 9 (*tpl* gene of *Serratia plymuthica*)

atgtcctacccagctgaaccttttcgcattaaaagcgttgaaaccctgacactgctttccaaagaggaaagagt
aaaaaaaataatatctgcaggctataatacatttctattgaattcacgggatgtttatattgatctgctgacag
acagcggcactaacgccatgagcgacaaacaatgggccggtataatgttgggtgatgaagcctatgccggcagt
gaaaatttctttcatcttgaaagtaccgtacaagaattatttggtttccggtatctggtccccactcatcaggg
gcgtggcgcagaaaacctgctctctcaggtagcgattacccccgggcagtttgtcgccggcaatatgtacttca
ccacaacccgctatcatcaggaaaaaaatggcgcggttttcgttgacgtcatcagagacgaagctcatgacgcc
agcgccattctgccgtttaaaggtgatattgacctgagcaagctagagacgctgatcgaggataaaggggcgaa
taacatcgcttatgtttgcctggcggtcaccgtcaatatggccggaggtcaaccggtctcgatgagtaacatgc
gcgccgtccgcgcactgacccaacggcatggcatcaaagtctattacgatgcgacccgctgcgttgaaaacgct
tacttcatcagagaaagagaagccggttacgaaaataccccgatttccgatatttttcatgaaatgtttagcta
tgccgacggctgcaccatgagcgggaaaaaagattgcctggtgaatatcggtggttttctgtgcatgaatgatg
agaagctttttggcgccgtacgggagctggttgtcgtctatgaaggcatgccgtcctatggcggcctggcaggg
cgcgatatggaagcgatggccataggtctgcgtgaggcgatgcagtttgaatatattgagcatcgcgtcaagca
agtgcgttatttaggcgataaattgaaagccgccggcattcccattgttgaacccgtgggtggacatgccgttt
tcctggatgcccgacggttttgcccacatctgcaacaggagcaattgccggcacaaagcctcgccgccagcatt
tacgtagagactggagtacgcgccatggaacgcggtattgtctcctcggggcgcaataaggagaccggtgaaaa
ctaccatcccaaacttgagacggtacgtctgacgataccgcgccgcgtatatacttacgcacatatggacaggg
tggccgatggcatcatcgaactgtttaaccggcgggaaaatatccgtggactgcgttttatttacgagcctaaa
caactgcgcttctttaccgcgcgctttgaatatttgtga

### Industrial Applicability

The transformant of the present invention can highly efficiently produce phenol using carbon dioxide as a sole carbon source, thus providing a solution to global warming caused by increased carbon dioxide emissions and contributing to high-efficient industrial production of phenol and chemical products produced using phenol as a raw material.

## Claims

1. A transformant obtained by introducing a tyrosine phenol-lyase gene described in (a), (b), (c), (d), or (e) below into a *Hydrogenophilus* bacterium.
(a) A DNA comprising the nucleotide sequence of SEQ ID NO: 2 or 4
(b) A DNA comprising a nucleotide sequence that has 90% or more identity to SEQ ID NO: 2 or 4 and encoding a polypeptide having tyrosine phenol-lyase activity
(c) A DNA encoding a polypeptide comprising the amino acid sequence of SEQ ID NO: 3 or 5
(d) A DNA encoding a polypeptide comprising an amino acid sequence that has 90% or more identity to SEQ ID NO: 3 or 5, the polypeptide having tyrosine phenol-lyase activity
(e) A DNA encoding a polypeptide comprising an amino acid sequence that has 1 to 40 amino acids deleted, substituted, inserted, or added in the amino acid sequence of SEQ ID NO: 3 or 5, the polypeptide having tyrosine phenol-lyase activity

2. The transformant according to claim 1, wherein the *Hydrogenophilus* bacterium is *Hydrogenophilus thermoluteolus*.

3. A method for producing phenol, the method comprising a step of culturing the transformant according to claim 1 or 2.
